# EUROPÄISCHE PATENTANMELDUNG

(11) **EP 0 876 811 A2**
(43) Veröffentlichungstag der Anmeldung: **11.11.1998**
(21) Anmeldenummer: 98105595.7
(22) Anmeldetag: 27.03.1998
(51) Int. Cl.: A61K 7/48, A61K 7/06

(54) **Vergällungsmittel für Ethanol**

(30) Priorität: 09.04.1997 DE 19714580
(71) Anmelder: HAARMANN & REIMER GMBH, D-37601 Holzminden (DE)
(72) Erfinder: Mansfeld, Gerd, 37632 Eschershausen (DE); Oelkers, Egon, 37639 Bevern (DE); Peters, Klaus, 37603 Holzminden (DE)
(74) Vertreter: Petrovicki, Wolfgang, Dr.

(57) **Zusammenfassung**

Die Erfindung betrifft ein Vergällungsmittel für Ethanol, Ethanol mit einem Gehalt dieses Vergällungsmittels und kosmetische Produkte mit einem Gehalt dieses vergällten Ethanols.

## Beschreibung

Die Erfindung betrifft ein Vergällungsmittel für Ethanol, Ethanol mit einem Gehalt dieses Vergällungsmittels und kosmetische Produkte mit einem Gehalt dieses vergällten Ethanols.

Ethylalkohol, der nicht für Lebensmittel und Genußmittel bestimmt ist, wie z.B. Ethylalkohol für die Herstellung von Kosmetika, ist aus steuerlichen Gründen durch Zusatz von sogenannten Vergällungsmitteln für den Genuß unbrauchbar zu machen. Im europäischen und außereuropäischen Raum werden zu diesem Zweck verschiedene Vergällungsmittel eingesetzt. Das weltweit am häufigsten eingesetzte Vergällungsmittel ist Diethylphthalat. In vielen Ländern werden auch natürliche etherische Öle und diverse einzelne Komponenten wie z.B. Isopropanol, Phenylethylalkohol, Moschus Keton usw. zur Vergällung verwendet.

Alle bekannten Vergällungsmittel weisen (teilweise erhebliche) Nachteile auf die die Produktqualität mindern. Diethylphthalat z.B. verursacht in Aerosolformulierungen Niesreiz und beeinflußt wie alle Einkomponenten-Zusätze den Geruchsverlauf von parfümierten Zusätzen in nicht kontrollierbarer Weise. Andere Vergällungsmittel wie z.B. Isopropanol, Moschus Keton beeinflussen den Geruchscharakter von parfümierten Produkten und sind teilweise (Moschus Keton) als farbinstabil bekannt.

Aufgabe der Erfindung war es, ein Vergällungsmittel zu entwickeln, das einen Vergällungseffekt etwa wie Diethylphthalat aufweist, aber die geschilderten Nachteile nicht besitzt; insbesondere sollte das neue Vergällungsmittel einen schwachen Eigengeruch aufweisen, der mit allen Parfumkompositionen harmoniert. Wir haben eine Zusammensetzung gefunden, die die gestellten Anforderungen erfüllt.

Gegenstand der Erfindung ist also eine Zusammensetzung enthaltend

| | | |
|---|---|---|
| A. | 50-250, vorzugsweise 80-120 | Gewichtsteile Benzylsalicylat, |
| B. | 5-40 | Gewichtsteile (i) 4.6.6.7.8.8.-Hexamethyl-1.3.4.6.7.8.-hexahydrocyclopenta[g]benzopyran oder (ii) Oxacyclohexadec-12- und/oder -13-en-2-on, jeweils gegebenenfalls gelöst in 5 bis 40 Gewichtsteilen (E und/oder F), |
| C. | 10-100, vorzugsweise 30-70 | Gewichtsteile hydrierter Wurzelöl-C₁-C₄-alkylester, vorzugsweise -methylester, |
| D. | 40-240, vorzugsweise 75-125 | Gewichtsteile 2-Pentylphenylpropanol, |
| E. | 200-500, vorzugsweise 250-350 | Gewichtsteile Benzylbenzoat, |
| F. | 100-400, vorzugsweise 150-250 | Gewichtsteile Dipropylenglykol und |
| G. | 150-300, vorzugsweise 150-250 | Gewichtsteile Triethylcitrat. |

Die Verbindung B (i) entspricht der Formel 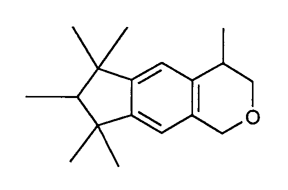 und wird von der Firma Bush Boake Allen/Großbritannien unter der Bezeichnung ®Abbalide und von der Firma International Flavors & Fragrances/USA unter der Bezeichnung ®Galaxolide vertrieben.

Die Komponente B (ii) wird als Isomerengemisch von uns selbst unter der Bezeichnung ®Globalid vertrieben.

Die Komponente C wird von der Firma Hercules/USA unter der Bezeichnung ®Hercolyn D-E vertrieben.

Die erfindungsgemäßen Zusammensetzungen lassen sich durch Mischen der einzelnen Komponenten herstellen. Für die Vergällung von Ethanol geeignete Konzentrationen der erfindungsgemäßen Zusammensetzungen liegen im Bereich von 0.01 bis 5, vorzugsweise 0.01 bis 3 kg auf 100 l Ethanol. Weiterer Gegenstand der Erfindung ist Ethanol mit einem Gehalt der beschriebenen Zusammensetzungen.

Ein weiterer Gegenstand der Erfindung sind mit dem erfindungsgemäß vergällten Ethanol hergestellte kosmetische Produkte. Diese Produkte können 1 bis 99 Gew.-%, bezogen auf Ethanol-haltiges kosmetisches Produkt, des vergällten Ethanols enthalten.

Derartige kosmetische Produkte umfassen (bevorzugter Ethanolgehalt in Klammern) Extrait, Eau de Parfum, Eau de Toilette, Eau de Cologne (jeweils 30 -99 Gew.-%), Splash Cologne, Rasierwasser, allgemeine Deo-Formulierungen, Haarfestiger, Haarspray, Haargele (1-50 Gew.-%), Gesichtswasser (5-70 Gew.-%), Körpergele, in besonderen Emulsionen (jeweils 1-40 Gew.-%), Haarwasser (10-75 Gew.-%), Handdesinfektionsmittel (20-99 Gew.-%), Glasreiniger (10-90 Gew.-%), Erfrischungstücher (30-90 Gew.-%) usw.

Die Teile und Prozentangaben der nachfolgenden Beispiele beziehen sich jeweils auf das Gewicht.

### Beispiele

### Beispiel 1

| **Teile** | **Stoff** |
|---|---|
| 100,00 | Benzylsalicylat |
| 50,00 | Chromanolid, 50 %-ig in Benzylbenzoat |
| 50,00 | Hercolyn D-E |
| 100,00 | 2-Pentylphenylpropanol |
| 300,00 | Benzylbenzoat |
| 200,00 | Dipropylenglykol |
| 200,00 | Triethylcitrat |

700 g der obigen Zusammensetzung werden mit 100 l Ethanol gemischt. Das so erhaltene vergällte Ethanol eignet sich hervorragend zur Herstellung von kosmetischen Produkten.

### Beispiel 2

| **Teile** | **Stoff** |
|---|---|
| 100,00 | Benzylsalicylat |
| 50,00 | Globalid, 50 %-ig in Dipropylenglykol |
| 50,00 | Herolyn D-E |
| 100,00 | 2-Pentylphenylpropanol |
| 300,00 | Benzylbenzoat |
| 200,00 | Dipropylenglykol |
| 200,00 | Triethylcitrat |

700 g der obigen Zusammensetzung werden mit 100 l Ethanol gemischt. Das so erhaltene vergällte Ethanol eignet sich hervorragend zur Herstellung von kosmetischen Produkten.

## Patentansprüche

1. Zusammensetzung enthaltend
| | | |
|---|---|---|
| A. | 50-250 | Gewichtsteile Benzylsalicylat, |
| B. | 5-40 | Gewichtsteile (i) 4.6.6.7.8.8.-Hexamethyl-1.3.4.6.7.8.-hexahydrocyclopenta[g]benzopyran oder (ii) Oxacyclohexadec-12- und/oder -13-en-2-on, jeweils gegebenenfalls gelöst in 5 bis 40 Gewichtsteilen (E und/oder F), |
| C. | 10-100 | Gewichtsteile hydrierter Wurzelöl-C₁-C₄-alkylester, |
| D. | 40-240 | Gewichtsteile 2-Pentylphenylpropanol, |
| E. | 200-500 | Gewichtsteile Benzylbenzoat, |
| F. | 100-400 | Gewichtsteile Dipropylenglykol und |
| G. | 150-300 | Gewichtsteile Triethylcitrat. |

2. Ethanol, enthaltend 0,01 bis 5 kg der Zusammensetzung nach Anspruch 1 pro 100 l Ethanol.

3. Ethanol, enthaltend 0,01 bis 3 kg der Zusammensetzung nach Anspruch 1 pro 100 l Ethanol.

4. Kosmetische Produkte mit einem Gehalt an Ethanol nach Ansprüchen 2 oder 3.
